# EUROPEAN PATENT APPLICATION

(11) **EP 2 092 886 A1**
(43) Date of publication of application: **26.08.2009**
(21) Application number: 07747897.2
(22) Date of filing: 16.04.2007
(51) Int. Cl.: A61B 5/117

(54) **DEVICE FOR PRODUCING AN IRIS IMAGE**

(30) Priority: 28.11.2006 RU 2006141885
(71) Applicant: Antonov, Dmitry Evgenievich, Moscow 127018 (RU)
(72) Inventor: Antonov, Dmitry Evgenievich, Moscow 127018 (RU)
(74) Representative: Andrae, Steffen
(86) International application number: PCT/RU2007/000185
(87) International publication number: WO 2008/066410

(57) **Abstract**

The present invention is aimed at simplifying the structure, operating conditions and ensuring a required placement of an individual to be identified in the focal plane of the optical system, in order to obtain a sharp image of the iris. The above object has been achieved by the fact that a device for obtaining an iris image comprises an image recording means 1 with a processing unit 2, an optical projection system 3 and a number of figures and/or bodies 4 placed in series along the axis of said system, wherein said figures and/ or bodies 4 being made and installed such that their images are projected on a focal point of the optical system in the form of a common matched figure. In doing so, said figures and/or bodies 4 may be made of the same or different shape such that a single image from the totality of separate bodies and figures is formed at the focus of the optical system. Moreover, said figures and/or bodies 4 may be made luminous or illuminated.

## Description

### FIELD OF THE INVENTION

The present invention relates to the procedure for protecting various objects against access of unauthorized persons through identification of an individual on an iris image thereof and may be used in diagnosis of the state of organs and functional systems of the organism using the iris.

### BACKGROUND OF THE INVENTION

There have been known various processes and devices to identify a human being on the totality of particular characteristic features present on the iris (see, US Patent No. 4641349).

There has been known an individual identifying device using the iris thereof comprising a means for recording the iris in the form of a wide angle camera and a unit for processing the data so obtained, said unit being connected to said camera (see, JP 10137223). The data processing unit is connected to a means for transmitting information to a human being whose personality is to be identified. These means is made in the form of a speaker that informs an individual to be identified of the necessity to change his or her location to obtain an iris image that is optimal for recognition.

The drawback to the known system resides in its relative complexity consisting in that the system for obtaining information on the iris should analyze it sufficiently quick, reveal defects (for example, as to the image sharpness) and, through a sufficiently complicated system of audible warning, give an indication to an individual to be identified of the necessity of one or other movements relative to the iris recording means.

The closest prior art with respect to the present invention has been disclosed in an individual identifying device using the iris thereof, comprising recording means in the form of a TV camera and a means for illuminating the iris of an individual to be identified made in the form of a laser (see, JP 10137220). In order to obtain a high-quality iris image, the system forms a preliminary iris image and, if the iris analysis results in the establishment of non-optimal location of an individual to be identified which is manifested in a diffuse image of the iris, the system generates a control action on the optical system that changes a focal distance and gains more sharp iris image.

The drawback to this system resides in its complexity, since a means for focusing onto the iris is required. By virtue of the fact that an individual to be identified locates in the process for "adjusting" the optical system at an indistinct fixed position and performs some movements relative to a selected position, this results in the fact that the procedure for focusing the optical system onto the iris of an individual to be identified with the aim of achieving an optimal image may take a good deal of time.

### SUMMARY OF THE INVENTION

It is an object of the present invention to simplify the structure, operating conditions and ensure a required placement of an individual to be identified in the focal plane of the optical system, in order to obtain a sharp image of the iris.

The above object has been achieved by the fact that a device for obtaining an iris image comprises an image recording means with a processing unit, an optical projection system and a number of figures and/ or bodies placed in series along the axis of said system; in doing so, said figures and/ or bodies are made and installed such that their images are projected on a focal point of the optical system in the form of a common matched figure.

The above object has also been achieved by the fact that said figures and/or bodies are made of the same shape.

The above object has also been achieved by the fact that said figures and/or bodies are made of different shape.

The above object has also been achieved by the fact that each image of a plane figure projecting on a focal point of the optical system in the form of a single common figure is formed from the totality of separate bodies and/or figures.

The above object has also been achieved by the fact that said figures and/or bodies are made luminous or illuminated.

The above object has also been achieved by the fact that said figures and/or bodies are rigidly coupled with each other.

The above object has also been achieved by the fact that said figures and/or bodies are rigidly coupled with each other, and their coupling is made luminous or illuminated.

The above object has also been achieved by the fact that said image recording means is made in the form of a CCD array or CMOS (complementary metal-oxide semiconductor) or another element that makes it possible to record an iris image.

### BRIEF DESCRIPTION OF THE DRAWINGS

In the drawings:
FIG. 1 illustrates in the most general form a principle scheme of a device for recording the iris using figures on glass plates.

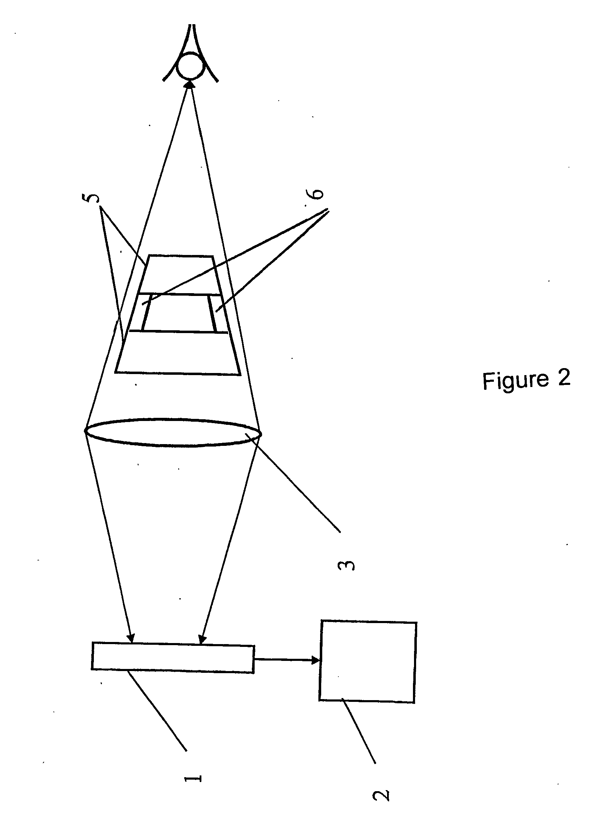

FIG. 2 illustrates in the most general form a principle scheme of a device for recording the iris using three-dimensional bodies.
FIGS. 3 and 4 illustrate variants of projections of geometrical figure images which the user must bring into coincidence or plot in a visual field of the projection optical system to locate his or her eye at its focus.

### BEST MODES FOR CARRYING OUT THE INVENTION

Accomplishment of a device for obtaining an iris image in the form of an image recording means with a processing unit, an optical projection system, and in-series arrangement of a number of figures, said figures being made and installed such that their images are projected on a focal point (focal plane) of the optical system in the form of a common matched figure, enable one to achieve the above-mentioned objects in the simplest manner. In fact, in the proposed device arrangement of a number of figures is such that if an individual to be identified appears in a visual field of the optical system such that figures to be observed match, this will mean that this individual is at the focus of the optical projection system and thus affords high quality of his iris image to be recorded. To the contrary, in order to ensure this high quality, an individual should locate in a visual field of the optical projection system such that he or she can observe a required image, for example, in the form of a single common matched figure which may be composed of several parts or figures located some distance apart.

The simplest embodiment seems to be that when these figures are made of the same geometrical shape, but varying in size. In so doing, the ratio of sizes and a distance at which these figures will be located from each other are chosen based upon a focal distance of the optical system in a visual field of which these figures rather than an individual to be identified are located. For instance, such figures may be two circles, two squares, two triangles etc. located symmetrically about an optical axis of the system. In such a case, if an individual moving in the area of the optical system focal plane, at some point in time brings an image of two non-equivalent circles into coincidence with one, this will mean that his or her eye is precisely at the foal point of the optical system and iris - in its focal plane.

In specific embodiments, use may be made of different figures, for example a circle and square, triangle and square or two halves of the circle, two halves of the square. In this case, to locate his or her eye at the focal point of the optical projection system, an individual must, e.g. bring two halves of the circle into coincidence with one, or two halves of the square with one, or, for instance, such that the circle be located inside the triangle or the triangle - inside the circle, etc.

In yet another specific embodiments, several isolated figures may be located in a visual field of the optical projection system; said figures, if an observer is located in a focal point of the optical projection system, will be observed as a single figure, for example, these may be four right angles, which with the correct look at them will form a square, or these may be three lines, which will be non-uniformly distant from an optical system focus and have different sizes, but when looking at them from the focal point, they will form a regular triangle.

For ease of adjustment processes, all the above-mentioned figures or their elements may be made in the form of two-dimensional images applied to glass plates which are located some distance apart in the optical channel of the projection system. In this case, however, there occur spurious glares and flare spots affecting the quality of an iris image to be recorded. Therefore it is advisable to make figures in the form of three-dimensional elements, for example, from wire, rods, etc. and rigidly couple them between each other.

In order for an individual to be identified to find easily a focal point of the projection system by correctly forming a required figure, it is advisable to make elements of the figures luminous or illuminated. And then an individual, while discerning them easily, may comply with requirements to be imposed upon him or her.

If geometrical figures are rigidly coupled with each other and the coupling itself is made luminous, a scenario of positioning becomes possible, when a human being gains minimization of luminescence of a coupling which is screened by a frontal figure. And if geometrical figures are rigidly coupled with each other and the coupling itself is made illuminated, a scenario of positioning becomes possible, when a human being gains a correct luminescence of illumination background (for example, a number of truncated cones inserted into each other to direct light to the focal point).

As recording means, use may be made of any known means, but in order a fixed image be easily digitized and subjected to processing (analysis, encoding, recognition) by means of a computer, it is advisable to make use of CCD or CMOS.

The essence of the claimed device is explained by embodiments thereof.

Example 1. In the most general case, a device for obtaining an iris image comprises an image recording means 1, which may be made, for example, in the form of a CCD array (for instance, a 1/1.8" CCD array, 2592x1944 pixels). The means 1 is connected to a unit 2 for processing data to be obtained. As such, it may be embodied in the form a personal computer equipped with relevant software. The means 1 is in a visual field of an optical projection system 3 that plots the image of an iris to be recorded in the plane of photosensitive elements of the CCD array. In a visual field of the projection system, between the CCD array and eye of an individual to be identified, there are located figures or bodies 4 placed in series, sizes and locations of said figures or bodies being chosen such that their image is projected on a focal point or focal plane of the optical system in the form of a common matched figure. In doing so, said figures may be made, for example, in the form of luminous lines on glasses or in the form of rods, bars, which may be illuminated by means of separate light sources (not shown in the drawings) that are not visible to an observer's eye but illuminate the figures.

A device is used as follows. An individual to be identified is located in a visual field of the optical projection system 3 and moves until he or she sees in a visual field of the system a required figure (depending on a device modification). For instance, it may be required that two circles of different diameter located in the optical channel, one of which is illuminated, e.g. with green color and the other - with red color, would be matched into one circle, or, e.g. two semicircles of different radius of curvature located with their concavities face-to-face would form one common circle in an individual's visual field. In a similar way, one may strive for matching two triangles, squares etc. into one, or halves of triangles, halves or quarters of squares or rectangles etc. into one. Then, with due regard for the selected ratios of sizes of elements to be matched and their mutual arrangement between each other, the eye of an individual to be identified will automatically be located in the focal plane of the projection system thus affording a high quality of an iris image to be recorded.

Example 2. A device for obtaining an iris image comprises an image recording means 1 made in the form of a 1/1.8" CCD array, 2592x1944 pixels. The means 1 is connected to a unit 2 for processing data to be obtained, that is, a personal computer equipped with relevant software. The means 1 is in a visual field of an optical projection system 3 that plots the image of an iris to be recorded in the plane of photosensitive elements of the CCD array. In a visual field of the projection system, between the CCD array and eye of an individual to be identified, there are located images of the halves of an equilateral triangle 4 as imaged on glass plates, said images being illuminated by a light source located out of an individual's visual field and the CCD array (not shown in the drawing). In doing so, the image of a triangle half of a greater diameter is located closer to the optical projection system and that of a lesser diameter - closer to the eye of an individual to be identified. The sizes of the triangle halves and the location of glass plates on which they are imaged are chosen such that the image of both halves is projected at the focus of the optical projection system in the form of one common matched figure - an equilateral triangle.

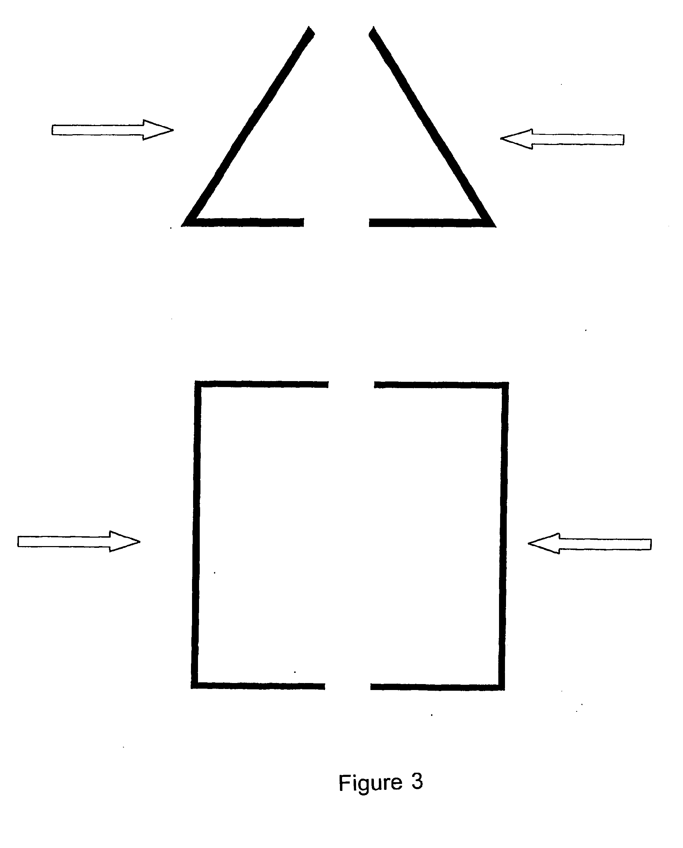

A device is used as follows. An individual to be identified is located in a visual field of the optical projection system 3 and moves until he or she sees in a visual field of the system an equilateral triangle image (see, Fig. 3). Then, with due regard for the selected ratios of sizes of elements to be matched and their mutual arrangement between each other, the eye of an individual to be identified will automatically be located in the focal plane of the projection system thus affording a high quality of an iris image to be recorded.

Example 3. A device for obtaining an iris image comprises an image recording means 1 made in the form of a 1/1.8" CCD array, 2592x1944 pixels. The means 1 is connected to a unit 2 for processing data to be obtained, that is, a personal computer equipped with relevant software. The means 1 is in a visual field of an optical projection system 3 that plots the image of an iris to be recorded in the plane of photosensitive elements of the CCD array. In a visual field of the projection system, between the CCD array and eye of an individual to be identified, there are located images of two halves of a square 4 as imaged on glass plates, said images being illuminated by a light source located out of an individual's visual field and the CCD array (not shown in the drawing). In doing so, the image of a square half of a greater diameter is located closer to the optical projection system and that of a lesser diameter - closer to the eye of an individual to be identified. The sizes of the square halves and the location of glass plates on which they are imaged are chosen such that the image of both halves is projected at the focus of the optical projection system in the form of one common matched figure - a square.

A device is used as follows. An individual to be identified is located in a visual field of the optical projection system 3 and moves until he or she sees in a visual field of the system a square image (see, Fig. 3). Then, with due regard for the selected ratios of sizes of elements to be matched and their mutual arrangement between each other, the eye of an individual to be identified will automatically be located in the focal plane of the projection system thus affording a high quality of an iris image to be recorded.

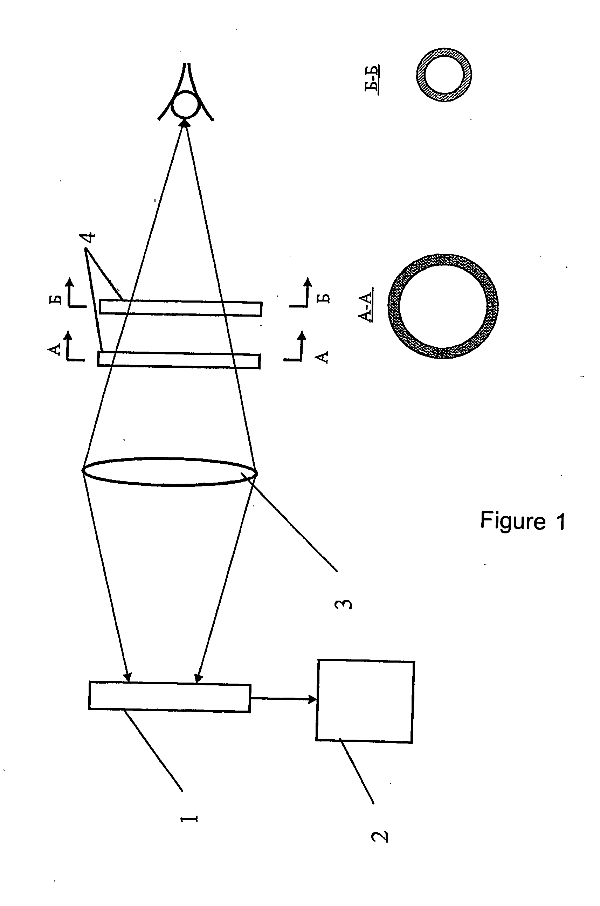

Example 4. A device for obtaining an iris image comprises an image recording means 1 made in the form of a 1/1.8" CCD array, 2592x1944 pixels. The means 1 is connected to a unit 2 for processing data to be obtained, that is, a personal computer equipped with relevant software. The means 1 is in a visual field of an optical projection system 3 that plots the image of an iris to be recorded in the plane of photosensitive elements of the CCD array. In a visual field of the projection system, between the CCD array and eye of an individual to be identified, there are located images of two circles 4 (see, Fig. 1) as imaged on glass plates, said images being illuminated by a light source located out of an individual's visual field and the CCD array (not shown in the drawing). In doing so, the image of a circle of a greater diameter is located closer to the optical projection system and that of a lesser diameter - closer to the eye of an individual to be identified. The sizes of the circles and the location of glass plates on which they are imaged are chosen such that the image of both circles is projected at the focus of the optical projection system in the form of one common matched circle.

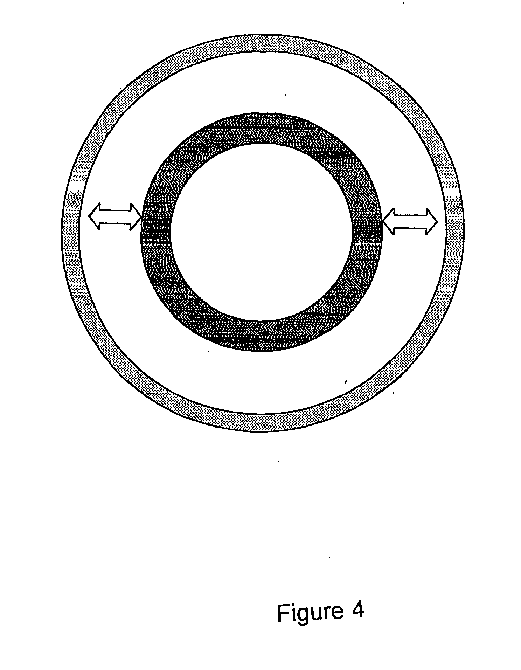

A device is used as follows. An individual to be identified is located in a visual field of the optical projection system 3 and moves until he or she sees in a visual field of the system an image of two, rather than one, circles (see, Fig. 4). Then, with due regard for the selected ratios of sizes of elements to be matched and their mutual arrangement between each other, the eye of an individual to be identified will automatically be located in the focal plane of the projection system thus affording a high quality of an iris image to be recorded.

Example 5. A device for obtaining an iris image comprises an image recording means 1 made in the form of a 1/1.8" CCD array, 2592x1944 pixels. The means 1 is connected to a unit 2 for processing data to be obtained, that is, a personal computer equipped with relevant software. The means 1 is in a visual field of an optical projection system 3 that plots the image of an iris to be recorded in the plane of photosensitive elements of the CCD array. In a visual field of the projection system, between the CCD array and eye of an individual to be identified, there are located two hollow truncated cones 5 with the same apex angle at the vertex, said cones being rigidly coupled between each other by means of two rods 6 which are parallel to generators of the cones with their end surfaces facing an individual's eye being illuminated by a light source located out of an individual's visual field and the CCD array (not shown in the drawing). In doing so, a hollow cone of a greater size is located closer to the optical projection system and that of a lesser size - closer to the eye of an individual to be identified. The sizes of hollow cones, their apex angle and location are chosen such that the image of both bases of the cones is projected at the focus of the optical projection system in the form of one common matched circle.

A device is used as follows. An individual to be identified is located in a visual field of the optical projection system 3 and moves until he or she sees in a visual field of the system an image of one circle (see, Fig. 4). In doing so, the aim of an individual to be identified is to achieve in a visual field of his or her eye an image of a ring of minimal width. Then, with due regard for the selected ratios of sizes of elements to be matched and their mutual arrangement between each other, the eye of an individual to be identified will be located, on its own, in the focal plane of the projection system thus affording a high quality of an iris image to be recorded.

Example 6. A device for obtaining an iris image comprises an image recording means 1 made in the form of a complementary metal-oxide semiconductor (CMOS-camera LZ0P396D). The means 1 is connected to a unit 2 for processing data to be obtained, that is, a personal computer equipped with relevant software. The means 1 is in a visual field of an optical projection system 3 that plots the image of an iris to be recorded in the plane of said complementary metal-oxide semiconductor (CMOS-camera LZ0P396D). In a visual field of the projection system, between the CMOS and eye of an individual to be identified, there are located two hollow truncated cones 5 with an the same apex angle at the vertex, said cones being rigidly coupled between each other by means of two rods 6 which are parallel to generators of the cones. These two rods are illuminated by a light source located out of an individual's visual field and the CMOS (not shown in the drawing). In doing so, a hollow cone of a greater size is located closer to the optical projection system and that of a lesser size - closer to the eye of an individual to be identified. The sizes of hollow cones, their apex angle and location are chosen such that the image of both bases of the cones is projected at the focus of the optical projection system in the form of one common matched circle.

A device is used as follows. An individual to be identified is located in a visual field of the optical projection system 3 and moves until he or she sees in a visual field of the system an image of one circle with illuminated coupling elements invisible. Then, with due regard for the selected ratios of sizes of elements to be matched and their mutual arrangement between each other, the eye of an individual to be identified will be located, on its own, in the focal plane of the projection system thus affording a high quality of an iris image to be recorded.

## Claims

1. A device for obtaining an iris image comprising an image recording means (1) with a processing unit (2), an optical projection system (3) and a number of figures and/or bodies (4) placed in series along the axis of said system, wherein said figures and/or bodies (4) being made and installed such that their images are projected on a focal point of the optical system in the form of a common matched figure.

2. A device as defined in claim 1, ***characterized in that*** said figures and/ or bodies (4) are made of the same shape.

3. A device as defined in claim 1, ***characterized in that*** said figures and/or bodies (4) are made of different shape.

4. A device as defined in claim 1, ***characterized in that*** each image of a plane figure projecting on a focal point of the optical system in the form of a single common figure is formed from the totality of separate figures and/or bodies (4).

5. A device as defined in claim 1, ***characterized in that*** said figures and/or bodies (4) are made luminous or illuminated.

6. A device as defined in claim 1, ***characterized in that*** said figures and/ or bodies (4) are rigidly coupled with each other.

7. A device as defined in claim 1, ***characterized in that*** said figures and/or bodies (4) are rigidly coupled with each other, and their coupling is made luminous or illuminated.

8. A device as defined in claim 1, ***characterized in that*** said image recording means (1) is made in the form of a CCD array or complementary metal-oxide semiconductor.
